# EUROPEAN PATENT SPECIFICATION

(11) **EP 4 368 171 B1**
(45) Date of publication and mention of the grant of the patent: **14.05.2025**
(21) Application number: 22383075.3
(22) Date of filing: 08.11.2022
(51) Int. Cl.: A61K 9/08, A61K 47/10, A61K 47/38, A61K 31/167, A61P 29/00

(54) **PARACETAMOL ORAL SOLUTION**
ORALE PARACETAMOLLÖSUNG
SOLUTION ORALE DE PARACETAMOL

(43) Date of publication of application: 15.05.2024
(73) Proprietor: LABORATORIOS ERN S.A., 08020 Barcelona (ES)
(72) Inventor: Solanes Valero, Irene, Barcelona (ES)
(74) Representative: Ponti & Partners, S.L.P

(56) References cited:
- EP-A2- 1 438 026
- GR-B- 1 009 462
- US-A- 6 102 254

## Description

### Technical field

The present invention relates to a paracetamol pharmaceutical composition in the form of oral solution, which is particularly effective for masking the unpleasant bitter taste of paracetamol.

### State of the art

Paracetamol is a common analgesic and antipyretic drug, which is considered as first-line therapy for the management of fever and mild-to-moderate pain, also in paediatrics.

Liquid oral formulations of paracetamol are recommended for paediatric use, because they are easy to administer and because they allow convenient selection of the correct dose for each particular paediatric patient.

Liquid oral formulations in the form of oral solutions are particularly suitable, as they ensure a complete active ingredient uniformity and, therefore, an accurate dosing, as well as a quick absorption. However, paracetamol has an unpleasant bitter taste, which needs to be masked in order to be acceptable by children and thus to provide its therapeutic value for the paediatric population. Taste masking of paracetamol oral solutions may be challenging, particularly for formulations comprising high concentration of paracetamol. Furthermore, other formulation requirements must be fulfilled, such as the physic and chemical stability, microbiological integrity, as well as a suitable viscosity to allow convenient dosage of the formulation.

Several proposals have been disclosed in the art for the formulation of taste-masked paracetamol oral solutions.

On the one hand, several formulations are available in the market for high-concentration paracetamol solutions. For example, the commercial product Apiretal^{®} 100 mg/ml is a paracetamol aqueous solution comprising polietilenglicol 600 and glycerol as cosolvents and sodium saccharin as sweetener, as well as flavouring and colouring agents.

Also, different proposals have been disclosed in the prior art. For example, the international patent application WO-A-2010/040652 discloses sugar free aqueous paracetamol solutions comprising a solubilizing agent containing polyethylene glycol, a thickening agent containing xanthan gum, and a sweetening system containing sucralose and a mixture of polyols including glycerol, sorbitol and xylitol. The polyethylene glycol used as solubilizing agent has preferably a high molecular weight, greater than 1000, typically, PEG 4000 or PEG 6000.

The international patent application WO-A-2013/147921 discloses liquid high-concentration paracetamol formulations. The formulations comprise a solvent system for dissolving paracetamol, which contains polyvinylpyrrolidone, polyethylene glycol, water and, optionally, other solvents such as propylene glycol, polysorbate 80 and a sugar alcohol, such as glycerol or sorbitol.

The international patent application WO-A-96/23486 discloses pharmaceutically acceptable taste masked liquid formulations comprising releatively large amount of unpleasant tasting medicines, wherein the taste masking effect is based on the use of a high-weight polyethylene glycol, of molecular weight of about 950-2000, which is melted before being added to the formulation.

The international patent application WO-A-03/034001 discloses paracetalmol taste-masked semi-solid gel compositions, which are based on the use of a carbomer as gelling agent, as it provides high yield value and shear thinning quality to the gel composition.

The European patent application EP-A-0614659 discloses pharmaceutical compositions in semisolid form and a device for their administration comprising a squeezable container. For example, a semisolid paracetamol formulation having a viscosity of 15000 cps is disclosed, comprising 3.2% paracetamol, which is thickened with sodium carboxymethyl cellulose.

Despite the different proposals available in the art, there is still the need for further high-concentration formulations of paracetamol in the form of solution, which have improved organoleptical properties for effectively masking the bitter taste of paracetamol.

### Object of the invention

The object of the invention is a paracetamol oral solution.

Another aspect of the invention is the paracetamol oral solution for use in therapy.

### Brief description of drawings

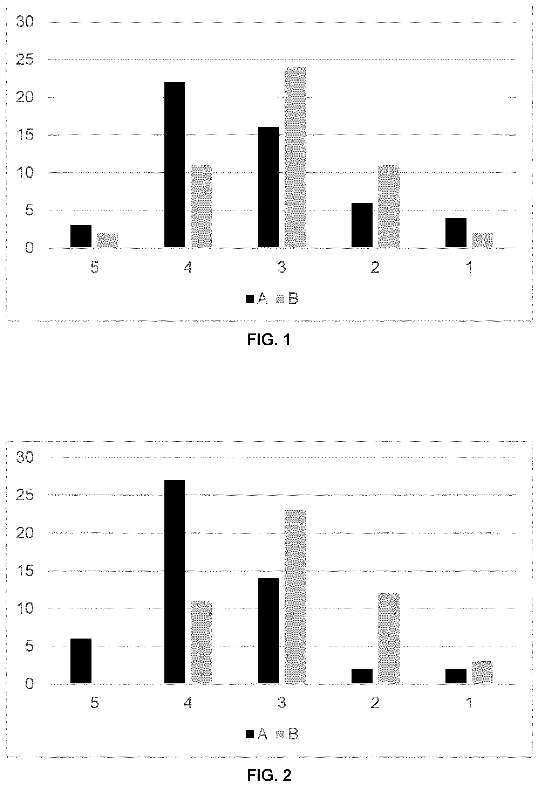

Figure 1 shows the result of the "taste/texture" assessment in the palatability assay disclosed in Example 3 comparing a paracetamol oral solution according to the invention (A) and the comparative commercial product Apiretal^{®} 100 mg/ml oral solution (B). The x-axis represents the 5-point assessment scale: 5 (very good), 4 (good), 3 (average), 2 (bad) and 1 (very bad); and the y-axis represents the number of subjects.
Figure 2 shows the result of the "aftertaste" assessment in the palatability assay disclosed in Example 3 comparing a paracetamol oral solution according to the invention (A) and the comparative commercial product Apiretal^{®} 100 mg/ml oral solution (B). The x-axis represents the 5-point assessment scale: 5 (very good), 4 (good), 3 (average), 2 (bad) and 1 (very bad); and the y-axis represents the number of subjects.

### Detailed description of the invention

The object of the present invention is a liquid pharmaceutical oral solution, which comprises:
a) paracetamol in an amount comprised between 85 mg/ml and 120 mg/ml;
b) low-weight polyethylene glycol;
c) glycerin;
d) carboxymethyl cellulose sodium; and
e) water,
wherein the pharmaceutical oral solution has a weight ratio of low-weight polyethylene glycol plus glycerine to water comprised between 2:1 and 8:1, and a weight ratio of glycerine to low-weight polyethylene glycol comprised between 1:1 and 2:1.

The authors of the present invention have developed a paracetamol liquid pharmaceutical composition in the form of aqueous solution, wherein, thanks to the use of low-weight polyethylene glycol and glycerin as solubilizing substances, combined with sodium carboxymethyl cellulose as viscosizing agent, provides a slightly viscose solution which is easily dispensable and effectively masks the bitter taste of paracetamol.

The concentration of the ingredients in the paracetamol oral solution are generally expressed herein as mg/ml, meaning milligrams of the specified component per millilitre of the final oral paracetamol solution.

The excipients used for preparing the composition of the present invention are well known in the art, and are widely available, and are described, for example, in the reference book P.J. Sheskey, W.G. Cook and C.G. Cable, Handbook of Pharmaceutical Excipients, Eighth Edition, Pharmaceutical Press, 2017 [ISBN: 978-0-8571-1271-2]. Also, common excipients and procedures for preparing the compositions are described in the book J.P Remington and A. R. Genaro, Remington, The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 4th edition, Churchill Livingstone Elsevier, 2013 [ISBN: 978-0-7020-4290-4].

### Paracetamol

Paracetamol is a well-known analgesic and anti-pyretic drug, also known as acetaminophen or as *N*-acetyl-para-aminophenol (CAS number 103-90-2). It is an odourless white crystalline solid of bitter taste.

The amount of paracetamol in the oral solution according to the present invention is comprised in the range 85-120 mg/ml, i.e., expressed as milligrams of paracetamol per millilitre of solution. Preferably, it is comprised between 90 mg/ml and 110 mg/ml, more preferably comprised between 95 mg/ml and 105 mg/ml and still more preferably is about 100 mg/ml.

### Solubilizing agents

Paracetamol is poorly soluble in water, with a reported solubility of less than 15 mg/g in water at 25°C (e.g., as reported in Granberg et al., Solubility of paracetamol in pure solvents, J. Chem. Eng. Data, 1999, 44, 1391-1395).

The paracetamol oral solution according to the present invention comprises low-weight polyethylene glycol and glycerine, which act as solubilizers for paracetamol. Both are well-known pharmaceutical excipients.

As is well-known, polyethylene glycol, also known as macrogol, is prepared by polymerization of ethylene oxide and is commercially available over a wide range of molecular weights, depending on the number of oxyethylene groups.

Low-weight polyethylene glycol, as used herein, typically means a polyethylene glycol having an average molecular weight comprised between about 400 and 900. Typically, the polyethylene glycol is selected from PEG 400, PEG 500, PEG 600, PEG, PEG 700, PEG 800, PEG 900, and mixtures thereof, preferably is selected from PEG 400, PEG 500, PEG 600, PEG 700, PEG 800, and mixtures thereof, more preferably is selected from PEG 500, PEG 600, PEG 700 and mixtures thereof, and still more preferably is PEG 600.

The polyethylene glycols of said molecular weight ranges are characterized in that they are liquid at room temperature (i.e. at about 25°C). Therefore, alternatively, low-weight polyethylene glycol, as used herein, also means a polyethylene glycol which is liquid at room temperature.

In an embodiment, the polyethylene glycol is PEG 600.

Generally, the total content of solubilizing agents, i.e., of polyethylene glycol plus glycerine, in the paracetamol solution amounts to from about 700 mg/ml to about 1100 mg/ml, preferably from 750 mg/ml to 1000 mg/ml, more preferably from 800 mg/ml to 950 mg/ml and still more preferably from 850 mg/ml to 900 mg/ml.

The weight ratio glycerin:polyethylene glycol is generally comprised between 1:1 and 2:1, preferably comprised between 1.1:1 and 1.8:1, more preferably comprised between 1.2:1 and 1.6:1, still more preferably comprised between 1.3:1 and 1.5:1, and still more preferably is about 1.4:1.

### Carboxymethylcellulose sodium

Carboxymethylcellulose sodium (NaCMC), also known as carmellose sodium is used in the paracetamol oral solution according to the present invention, as viscosizing agent.

Carboxymethylcellulose sodium is a well-known excipient, which is widely available from different commercial suppliers, for example, from the company Ashland under the trademarks Aqualon^{®} or Blanose^{®}; or from Nouryon under the trademark Akucell^{®}, among many others.

It is available in different grades, typically classified as being of low, medium or high viscosity, depending on the average molecular weight of the NaCMC and its degree of substitution.

All types and grades of NaCMC are suitable to be used in the present composition. Preferred is low and medium viscosity NaCMC, typically providing viscosities approximately comprised between 25 and 2500 mPa·s in aqueous solution at 2% w/v, measured at about 20°C. More preferred is a NaCMC providing viscosity comprised between 400 mPa·s and 800 mPa·s, more preferably comprised between 400 mPa·s and 600 mPa·s in aqueous solution at 2% w/v, measured at about 20°C. The viscosity can be measured as described in the monograph of carboxymethylcellulose sodium of the European Pharmacopoeia 11.0.

The amount of NaCMC is adjusted accordingly to provide a suitable viscosity to the final formulation. Typically, the viscosity of the paracetamol solution according to the invention is comprised between about 3000 mPa·s and about 7000 mPa·s, preferably comprised between about 3500 mPa·s and about 6000 mPa·s, measured at about 25°C.

The viscosity measurements can be made using a Brookfield Viscometer.

In one embodiment, the amount of NaCMC in the composition is comprised between 2 mg/ml and 25 mg/ml, preferably comprised between 4 mg/ml and 20 mg/ml, more preferably comprised between 6 mg/ml and 14 mg/ml, for example, about 6, 7, 8, 9, 10, 11, 12, 13 or 14 mg/ml.

### Additional optional ingredients

### Sweetening agents

Optionally, the composition may comprise a sweetening agent to improve the palatability of the paracetamol solution.

Typically, a non-nutritive high-intensity sweetener is used, for example, selected from aspartame, acesulfame potassium, alitame, neohesperidin dihydrochalcone, neotame, saccharin, saccharin sodium, sucralose, thaumatin, and mixtures thereof. Preferably, the sweetening agent is selected from saccharin, saccharin sodium, sucralose and neohesperidin, and mixtures thereof, and more preferably is selected from sucralose, neohesperidin, and mixtures thereof.

The amount of the sweetening agent may widely vary depending on the sweetening intensity of the agent, but generally is comprised in the range 0.001 mg/ml to 10 mg/ml, preferably from 0.1 mg/ml to 10 mg/ml.

In a preferred embodiment, the sweeter agent is a mixture of sucralose and neohesperidin. According to this embodiment, sucralose is typically added in a concentration of from about 1 mg/ml to 10 mg/ml, preferably from 2 mg/ml to 8 mg/ml, more preferably from 4 mg/ml to 6 mg/ml, and still more preferably in a concentration of about 5 mg/ml; and neohesperidin is typically added in a concentration in the range 0.1 mg/ml to 1.5 mg/ml, preferably 0.4 mg/ml to 1 mg/ml.

### Flavouring agents

The composition may optionally additionally comprise a flavouring agent for providing a pleasant flavour and/or odour to the composition. Suitable flavouring agents include natural and artificial flavours. Some suitable flavours are, for example, menthol, cinnamon, clove, anise, eucalyptus, peppermint, spearmint, thyme, vanilla, chocolate, sugar flavour, fruit flavours, such as cherry flavour, grape flavour, orange flavour, banana flavour, strawberry flavour, lemon flavour, apple flavour, peach flavour, raspberry flavour, pineapple flavour and apricot flavour among many others, and combinations thereof.

A preferred flavouring agent is a combination of sugar flavour with a fruit flavour, for example, with cherry flavour, strawberry flavour, peach flavour, raspberry flavour or apricot flavour.

The amount of flavouring agent can be easily adjusted by the skilled formulator, depending on the specific flavouring agent and the desired organoleptic effect. Typically, the amount of flavouring agent, if present in the composition, ranges from about 0.01 mg/ml to about 25 mg/ml.

### Colouring agent

The paracetamol oral solution may also contain a colouring agent to improve its appearance and make the product more organoleptically appealing.

Any colouring agent suitable for use in foods or pharmaceuticals may be used, as are well known in the art, for example, those disclosed in the "Coloring Agents" section of the Handbook of Pharmaceutical Ingredients *op. cit.*

The selection of a suitable colouring agent and its appropriate concentration is routine for the skilled in pharmaceutical formulation.

Suitable colouring agents include, for example, curcumin (E100), riboflavin (E101), tartrazine (E102), quinoline yellow (E104), sunset yellow FCF (E110), carmine cochineal (E120), amaranth (E123), ponceau 4R (E124), erythrosine (E127), allura red AC (E129), indigo carmine (E132), clorophylls (E140), or caramel (E150), among many others.

### Preservative

The paracetamol oral solution according to the present invention comprises glycerol, which is also an antimicrobial preservative. Therefore, in general, there is no need of an additional preservative to ensure the protection of the composition against microbial contamination.

Optionally, an additional preservative may be added, for example, butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate or thimerosal, for example, among others, or mixtures thereof.

In the case that an additional preservative is used, the amount selected will be based on the specific preservative and typically may range from about 0.001 mg/ml to about 50 mg/ml.

### pH adjusting agent

The pH of the paracetamol solution according to the present invention is generally around neutrality, typically the pH value is comprised between 6.0 and 8.0, preferably comprised between 6.5 and 7.5.

In general, such pH is obtained without the need to use any pH adjuster. Optionally, if required, pH adjusting agent may be added in order to adjust the pH value to the preferred range. In such case, the amount and type of the pH adjusting agent to be used will be easily determined by the skilled in the art.

As is well-known in the art, the pH adjusting agent may be an acidifying agent, such as citric acid, acetic acid, hydrochloric acid, lactic acid, phosphoric acid, or sulfuric acid, for example, and/or an alkalinizing agent, such as ammonia solution, diethanolamine, monoethanolamine, potassium bicarbonate, sodium bicarbonate, potassium citrate, sodium citrate, sodium bicarbonate, or sodium borate, for example. Said acidifying and alkalinizing agents can be used in combination, as buffering system, generally comprising an acid and its conjugated base, as is well-known in the art.

### Compositions

Another component of the paracetamol oral solution is water, which is used as solvent. Typically, purified water for pharmaceutical use is used, which is available commercially, commonly obtained by distillation, ion exchange or reverse osmosis from drinkable water. The amount of water is typically adjusted to provide the desired concentration of every ingredient, as discussed above.

Generally, the weight ratio of the solubilizing agents, i.e., of polyethylene glycol plus glycerine, to water in the paracetamol solution is comprised between 2:1 and 8:1, preferably comprised between 3:1 and 7:1, more preferably comprised between 4:1 and 6:1, and still more preferably is about 5:1.

The present invention provides a pharmaceutical oral solution. The term solution is understood in its ordinary meaning as known in the art, i.e., a homogeneous, molecular, mixture of two or more components. In particular, the solid paracetamol, as well as the solid excipients of the formulation, are dissolved in the solvent system comprising the low-weight polyethylene glycol, glycerin and water. The term oral solution means that the paracetamol solution is for oral administration.

The paracetamol solution of the present invention can be prepared using standard procedures, by simple mixture of the components.

A clear solution is obtained by thoroughly mixing the above stated ingredients in a suitable vessel provided with a stirring system, such as a blade stirrer. In order to obtain an optimal and uniform viscosizing effect, the NaCMC is preferably previously dispersed, for example in glycerin, before being incorporated, typically as the last step of the process, followed by high shear mixing of the resulting solution.

The paracetamol oral solution of the invention can be filled into a standard multiple-dose primary container suitable for oral liquid pharmaceutical dosage forms, such glass or plastic bottle, typically provided with a suitable dose measure system, such as a graded vessel or spoon, or a syringe.

The paracetamol solution is a clear, homogeneous solution, slightly viscous, which can be easily dispensed according to the required dose.

As shown in the Examples, the solution is stable and has outstanding organoleptic properties. The comparative palatability assay disclosed in Example 3 shows that the paracetamol oral solution according to the present invention was found organoleptically superior, compared to a commercial paracetamol oral solution (Apiretal^{®} 100 mg/ml oral solution).

In view of these properties, the paracetamol oral solution is suitable to be used in therapy, particularly for treating the paediatric patients. In particular, the paracetamol oral solution can be used for the treatment of fever and mild-to-moderate pain.

Therefore, another aspect of the present invention is the paracetamol oral solution for use in therapy, in particular, for use in the treatment of fever and/or mild-to-moderate pain, preferably in the paediatric patient.

The paediatric patient, as used herein, typically includes children from 0 to about 10 years.

The skilled practitioner will have no difficulties in determining the most suitable dose, according to the common medical guidelines. Typically, paracetamol is administered to the paediatric patient in daily doses of about 60 mg/kg, distributed in 4-6 daily administrations.

In an embodiment, the paracetamol oral solution consists of:
a) paracetamol in an amount comprised between 85 mg/ml and 120 mg/ml;
b) low-weight polyethylene glycol;
c) glycerin;
d) carboxymethyl cellulose sodium;
e) water;
f) optionally, a sweetening agent;
g) optionally, a flavouring agent;
h) optionally a colouring agent;
i) optionally, a preservative; and
j) optionally, a pH adjusting agent.

In an embodiment, the paracetamol oral solution consists of:
a) paracetamol in an amount comprised between 85 mg/ml and 120 mg/ml;
b) low-weight polyethylene glycol;
c) glycerin;
d) carboxymethyl cellulose sodium;
e) water;
f) a sweetening agent;
g) a flavouring agent;
h) optionally a colouring agent;
i) optionally, a preservative; and
j) optionally, a pH adjusting agent.

In an embodiment, the paracetamol oral solution consists of:
a) paracetamol in an amount comprised between 85 mg/ml and 120 mg/ml;
b) low-weight polyethylene glycol;
c) glycerin;
d) carboxymethyl cellulose sodium;
e) water;
f) a sweetening agent;
g) a flavouring agent; and
h) optionally, a colouring agent.

The invention relates to the following embodiments:
1.- A pharmaceutical oral solution, which comprises:
   a) paracetamol in an amount comprised between 85 mg/ml and 120 mg/ml;
   b) low-weight polyethylene glycol;
   c) glycerin;
   d) carboxymethyl cellulose sodium; and
   e) water.
2.- The pharmaceutical oral solution according to embodiment 1, characterised in that it consists of:
   a) paracetamol in an amount comprised between 85 mg/ml and 120 mg/ml;
   b) low-weight polyethylene glycol;
   c) glycerin;
   d) carboxymethyl cellulose sodium;
   e) water;
   f) optionally, a sweetening agent;
   g) optionally, a flavouring agent;
   h) optionally, a colouring agent;
   i) optionally, a preservative; and
   j) optionally, a pH adjusting agent.
3.- The pharmaceutical oral solution according to embodiment 2, characterised in that it consists of:
   a) paracetamol in an amount comprised between 85 mg/ml and 120 mg/ml;
   b) low-weight polyethylene glycol;
   c) glycerin;
   d) carboxymethyl cellulose sodium;
   e) water;
   f) a sweetening agent;
   g) a flavouring agent;
   h) optionally a colouring agent;
   i) optionally, a preservative; and
   j) optionally, a pH adjusting agent.
4.- The pharmaceutical oral solution according to embodiment 3, characterized in that it consists of:
   a) paracetamol in an amount comprised between 85 mg/ml and 120 mg/ml;
   b) low-weight polyethylene glycol;
   c) glycerin;
   d) carboxymethyl cellulose sodium;
   e) water;
   f) a sweetening agent;
   g) a flavouring agent; and
   h) optionally, a colouring agent.
5.- The pharmaceutical oral solution according to any one of embodiments 1 to 4, characterized in that the amount of paracetamol is comprised between 90 mg/ml and 110 mg/ml, preferably comprised between 95 mg/ml and 105 mg/ml and still more preferably is about 100 mg/ml.
6.- The pharmaceutical oral solution according to any one of embodiments 1 to 5, characterized in that the low-weight polyethylene glycol has an average molecular weight comprised between 400 and 900.
7.- The pharmaceutical oral solution according to any one of embodiments 1 to 6, characterized in that the low-weight polyethylene glycol is selected from PEG 400, PEG 500, PEG 600, PEG, PEG 700, PEG 800, PEG 900, and mixtures thereof.
8.- The pharmaceutical oral solution according to any one of embodiments 1 to 7, characterized in that the low-weight polyethylene glycol is a polyethylene glycol which is liquid at room temperature.
9.- The pharmaceutical oral solution according to embodiments 6 to 8, characterized in that the low-weight polyethylene glycol is PEG 600.
10.- The pharmaceutical oral solution according to any one of embodiments 1 to 9, characterized in that the total content of low-weight polyethylene glycol plus glycerin amounts to from 700 mg/ml to 1100 mg/ml, preferably from 750 mg/ml to 1000 mg/ml, more preferably from 800 mg/ml to 950 mg/ml and still more preferably from 850 mg/ml to 900 mg/ml.
11.- The pharmaceutical oral solution according to any one of embodiments 1 to 10, characterized in that weight ratio glycerin: polyethylene glycol is comprised between 1:1 and 2:1, preferably comprised between 1.1:1 and 1.8:1, more preferably comprised between 1.2:1 and 1.6:1, still more preferably comprised between 1.3:1 and 1.5:1, and still more preferably is about 1.4:1.
12.- The pharmaceutical oral solution according to any one of embodiments 1 to 11, characterized in that it has a viscosity comprised between 3000 mPa·s and 7000 mPa·s, preferably comprised between 3500 mPa·s and 6000 mPa·s, measured at 25°C.
13.- The pharmaceutical oral solution according to any one of embodiments 1 to 12, characterized in that the grade of the carboxymethylcellulose sodium is such that it provides a viscosity comprised between 25 mPa·s and 2500 mPa·s, preferably comprised between 400 mPa·s and 800 mPa·s, and more preferably comprised between 400 mPa·s and 600 mPa·s in aqueous solution at 2% w/v, measured at 20°C.
14.- The pharmaceutical oral solution according to any one of embodiments 1 to 13, characterized in that the amount of carboxymethylcellulose sodium is comprised between 2 mg/ml and 25 mg/ml, preferably comprised between 4 mg/ml and 20 mg/ml, more preferably comprised between 6 mg/ml and 14 mg/ml.
15.- The pharmaceutical oral solution according to any one of embodiments 1 to 14, characterized in that the weight ratio of polyethylene glycol plus glycerine to water is comprised between 2:1 and 8:1, preferably comprised between 3:1 and 7:1, more preferably comprised between 4:1 and 6:1, and still more preferably is about 5:1.
16.- The pharmaceutical oral solution according to any one of embodiments 1 to 15, characterized in that the solution comprises a sweetening agent, which is preferably selected from aspartame, acesulfame potassium, alitame, neohesperidin dihydrochalcone, neotame, saccharin, saccharin sodium, sucralose, thaumatin, and mixtures thereof, more preferably is selected from saccharin, saccharin sodium, sucralose, neohesperidin, and mixtures thereof.
17.- The pharmaceutical oral solution according to embodiment 16, characterized in that the sweetening agent is a mixture of sucralose and neohesperidin.
18.- The pharmaceutical oral solution according to embodiment 17, characterized in that the amount of sucralose is comprised between 1 mg/ml and 10 mg/ml, preferably between 2 mg/ml and 8 mg/ml, more preferably between 4 mg/ml and 6 mg/ml, and still more preferably is about 5 mg/ml; and the amount of neohesperidin is comprised between 0.1 mg/ml and 1.5 mg/ml, preferably comprised between 0.4 mg/ml and 1 mg/ml.
19.- The pharmaceutical oral solution according to any one of embodiments 1 to 18, characterized in that it comprises a flavouring agent, preferably in an amount comprised between 0.01 mg/ml and 25 mg/ml.
20.- The pharmaceutical oral solution according to any one of embodiments 1 to 19 for use in therapy.
21.- The pharmaceutical oral solution for use according to embodiment 20, characterized in that it is for the treatment of fever and/or mild-to-moderate pain.
22.- The pharmaceutical oral solution for use according to embodiments 20 or 21, characterized in that it is for use in the paediatric patient.

### Examples

### Example 1: Paracetamol oral solution

A paracetamol solution according to the present invention, containing 100 mg/ml of paracetamol, was prepared with the ingredients disclosed in the following table:

| **Ingredient** | **mg/ml** |
|---|---|
| Paracetamol | 100.0 |
| PEG 600 | 364.5 |
| Glycerin | 506.3 |
| NaCMC | 8.0 |
| Sucralose | 5.0 |
| Neohesperidine | 0.8 |
| Flavouring agent | 20.0 |
| Colouring agent | 0.1 |
| Water | 174.3 |
| Total | 1179.1 |

The sodium carboxymethyl cellulose used was a low-medium viscosity carboxymethylcellulose that provides a viscosity between 400 mPa·s and 800 mPa·s (674 mPa·s) in aqueous solution at 2% w/v. It conforms to the monograph for carmellose sodium in the current European Pharmacopoeia.

The solution was prepared as follows. In a stainless-steel reactor equipped with agitation system, PEG 600 was first mixed with a small part of water (about 20% of total water), and paracetamol was then added and stirred until complete dissolution. The flavouring agents, sucralose, neohesperidin and colouring agent were subsequently added, dissolved in water, under continuous stirring. Sodium carboxymethylcellulose and glycerin were premixed in a separate vessel and added to the previous mix, the rest of water was added, to make up the final volume, and the obtained final mixture was maintained under high-shear mixing using a Cowles stirrer for about 60 minutes.

The obtained product was a clear red-coloured slightly viscous solution. The viscosity was 5340 mPa·s, measured at 25°C using a Visco Star Plus Viscometer, equipped with a number 3 spindle rotating at 20 rpm. The pH of the solution was 7.0.

### Example 2: Stability assay

The paracetamol composition prepared in Example 1 was tested for physicochemical and microbiological integrity in a 3-month stability assay, at 40 °C on the stove and at room temperature. At the end of this period, the product was tested and it was confirmed that all the required specifications (aspect, pH, viscosity, impurities, paracetamol concentration and microbiological integrity) were fulfilled.

### Example 3: Palatability study

The palatability of the composition of Example 1 was assessed, compared to the commercial product Apiretal^{®} 100 mg/ml oral solution. The excipients of this product are PEG 600 and glycerol as solubilizing substances, sodium saccharin as sweetener, flavouring agents and water.

A comparative single-blind palatability study was designed, with 51 adult volunteers aged from 25 to 60.

All participants tested both formulations, i.e., Example 1 according to the invention (formulation A), and comparative Apiretal^{®} 100 mg/ml oral solution (formulation B). They were randomly assigned a specific sequence, so each volunteer received the samples in a specified order. About half of volunteers (group 1, 25 subjects) received first the composition according to the invention, while the other half (group 2, 26 subjects) received first the comparative formulation. The preparations were administered approximately 5 min apart to allow time for taking and rating the preparation and cleaning the mouth.

Each volunteer was asked to rate the taste/texture of the compositions and their aftertaste using the following scale: "very good", "good", "average", "bad" and "very bad". They were also asked about their preference between both formulations.

The results of the preference selection between formulations are summarized in the following table:

| **Population** | **A preferred** | **B preferred** |
|---|---|---|
| Total group | 65% | 35% |
| Group 1 | 76% | 24% |
| Group 2 | 54% | 46% |

It was found that 65% of the volunteers preferred the composition according to the invention. It was observed that the administration order had an influence in the preference, as shown in the results for groups 1 and 2, but, nevertheless, a higher percentage for the composition of Example 1 was found in both groups.

The results of the taste/texture and aftertaste evaluation are shown in Figures 1 and 2, respectively. The rating scale numbering in the figures corresponds to: 5 (very good), 4 (good), 3 (average), 2 (bad) and 1 (very bad).

It could be appreciated better rating (5 and 4 scores) for the composition of the present invention (formulation A) in both tests.

Using Excel TTEST for calculating the probability associated with the t-student test, a statistically significative difference was found in favour of composition A regarding aftertaste (Figure 2). For group 1, statistically significative differences in favour of composition A were found for both taste/texture and aftertaste.

## Claims

1. A liquid pharmaceutical oral solution, which comprises:
a) paracetamol in an amount comprised between 85 mg/ml and 120 mg/ml;
b) low-weight polyethylene glycol;
c) glycerin;
d) carboxymethyl cellulose sodium; and
e) water,
wherein the pharmaceutical oral solution has a weight ratio of low-weight polyethylene glycol plus glycerine to water comprised between 2:1 and 8:1, and a weight ratio of glycerine to low-weight polyethylene glycol comprised between 1:1 and 2:1.

2. The pharmaceutical oral solution according to claim 1, **characterized in that** it consists of:
a) paracetamol in an amount comprised between 85 mg/ml and 120 mg/ml;
b) low-weight polyethylene glycol;
c) glycerin;
d) carboxymethyl cellulose sodium;
e) water;
f) a sweetening agent;
g) a flavouring agent; and
h) optionally, a colouring agent.

3. The pharmaceutical oral solution according to claims 1 or 2, **characterized in that** the amount of paracetamol is comprised between 90 mg/ml and 110 mg/ml, preferably comprised between 95 mg/ml and 105 mg/ml and still more preferably is about 100 mg/ml.

4. The pharmaceutical oral solution according to any one of claims 1 to 3, **characterized in that** the low-weight polyethylene glycol has an average molecular weight comprised between 400 and 900.

5. The pharmaceutical oral solution according to any one of claims 1 to 4, **characterized in that** the low-weight polyethylene glycol is selected from PEG 400, PEG 500, PEG 600, PEG, PEG 700, PEG 800, PEG 900, and mixtures thereof, and preferably is PEG 600.

6. The pharmaceutical oral solution according to any one of claims 1 to 5, **characterized in that** the total content of low-weight polyethylene glycol plus glycerin amounts to from 700 mg/ml to 1100 mg/ml, preferably from 750 mg/ml to 1000 mg/ml, more preferably from 800 mg/ml to 950 mg/ml and still more preferably from 850 mg/ml to 900 mg/ml.

7. The pharmaceutical oral solution according to any one of claims 1 to 6, **characterized in that** weight ratio glycerin:low-weight polyethylene glycol is comprised between 1.1:1 and 1.8:1, preferably comprised between 1.2:1 and 1.6:1, more preferably comprised between 1.3:1 and 1.5:1, and still more preferably is about 1.4:1.

8. The pharmaceutical oral solution according to any one of claims 1 to 7, **characterized in that** it has a viscosity comprised between 3000 mPa·s and 7000 mPa·s, preferably comprised between 3500 mPa·s and 6000 mPa·s, measured at 25°C.

9. The pharmaceutical oral solution according to any one of claims 1 to 8, **characterized in that** the grade of the carboxymethylcellulose sodium is such that it provides a viscosity comprised between 25 mPa·s and 2500 mPa·s, preferably comprised between 400 mPa·s and 800 mPa·s, and more preferably comprised between 400 mPa·s and 600 mPa·s in aqueous solution at 2% w/v, measured at 20°C.

10. The pharmaceutical oral solution according to any one of claims 1 to 9, **characterized in that** the amount of carboxymethylcellulose sodium is comprised between 2 mg/ml and 25 mg/ml, preferably comprised between 4 mg/ml and 20 mg/ml, more preferably comprised between 6 mg/ml and 14 mg/ml.

11. The pharmaceutical oral solution according to any one of claims 1 to 10, **characterized in that** the weight ratio of low-weight polyethylene glycol plus glycerine to water is comprised between 3:1 and 7:1, more preferably comprised between 4:1 and 6:1, and still more preferably is about 5:1.

12. The pharmaceutical oral solution according to any one of claims 1 to 11, **characterized in that** the solution comprises a sweetening agent, which is preferably selected from aspartame, acesulfame potassium, alitame, neohesperidin dihydrochalcone, neotame, saccharin, saccharin sodium, sucralose, thaumatin, and mixtures thereof, more preferably is selected from saccharin, saccharin sodium, sucralose, neohesperidin, and mixtures thereof, and more preferably is a mixture of sucralose and neohesperidin.

13. The pharmaceutical oral solution according to any one of claims 1 to 12, **characterized in that** it comprises a flavouring agent, preferably in an amount comprised between 0.01 mg/ml and 25 mg/ml.

14. A liquid pharmaceutical oral solution according to any one of claims 1 to 13 for use in therapy.

15. The pharmaceutical oral solution for use according to claim 14, **characterized in that** it is for the treatment of fever and/or mild-to-moderate pain, preferably in the paediatric patient.

## Patentansprüche

1. Flüssige, pharmazeutische orale Lösung, die umfasst:
a) Paracetamol in einer zwischen 85 mg/ml und 120 mg/ml liegenden Menge;
b) niedermolekulares Polyethylenglycol;
c) Glycerin;
d) Natriumcarboxymethylcellulose; und
e) Wasser,
wobei die pharmazeutische orale Lösung ein Gewichtsverhältnis von niedermolekularem Polyethylenglycol plus Glycerin zu Wasser, die zwischen 2:1 und 8:1 liegt, und ein Gewichtsverhältnis von Glycerin zu niedermolekularem Polyethylenglycol, die zwischen 1:1 und 2:1liegt, aufweist.

2. Pharmazeutische orale Lösung nach Anspruch 1, **dadurch gekennzeichnet, dass** sie besteht aus:
a) Paracetamol in einer zwischen 85 mg/ml und 120 mg/ml liegenden Menge;
b) niedermolekularem Polyethylenglycol;
c) Glycerin;
d) Natriumcarboxymethylcellulose;
e) Wasser;
f) einem Süßungsmittel;
g) einem Aromastoff; und
h) optional einem farbgebenden Mittel.

3. Pharmazeutische orale Lösung nach Anspruch 1 oder 2, **dadurch gekennzeichnet, dass** die Menge an Paracetamol zwischen 90 mg/ml und 110 mg/ml liegt, vorzugsweise zwischen 95 mg/ml und 105 mg/ml liegt und insbesondere etwa 100 mg/ml beträgt.

4. Pharmazeutische orale Lösung nach einem der Ansprüche 1 bis 3,
**dadurch gekennzeichnet, dass** das niedermolekulare Polyethylenglycol ein mittleres Molekulargewicht zwischen 400 und 900 aufweist.

5. Pharmazeutische orale Lösung nach einem der Ansprüche 1 bis 4,
**dadurch gekennzeichnet, dass** das niedermolekulare Polyethylenglycol ausgewählt ist aus PEG 400, PEG 500, PEG 600, PEG, PEG 700, PEG 800, PEG 900 und Gemischen davon, vorzugsweise PEG 600 ist.

6. Pharmazeutische orale Lösung nach einem der Ansprüche 1 bis 5,
**dadurch gekennzeichnet, dass** der Gesamtgehalt an niedermolekularem Polyethylenglycol plus Glycerin von 700 mg/ml bis 1100 mg/ml, vorzugsweise von 750 mg/ml bis 1000 mg/ml, insbesondere von 800 mg/ml bis 950 mg/ml und besonders bevorzugt von 850 mg/ml bis 900 mg/ml beträgt.

7. Pharmazeutische orale Lösung nach einem der Ansprüche 1 bis 6,
**dadurch gekennzeichnet, dass** das Gewichtsverhältnis Glycerin:
niedermoelkularem Polyethylenglykol zwischen 1:1 und 2:1 liegt, vorzugsweise zwischen 1,1:1 und 1,8:1 liegt, insbesondere zwischen 1,2:1 und 1,6:1 liegt, besonders bevorzugt zwischen 1,3:1 und 1,5:1 liegt, und ganz besonders bevorzugt bei etwa 1,4:1 liegt.

8. Pharmazeutische orale Lösung nach einem der Ansprüche 1 bis 7,
**dadurch gekennzeichnet, dass** sie eine zwischen 3000 mPa·s und 7000 mPa·s liegende, vorzugsweise zwischen 3500 mPa·s und 6000 mPa·s liegende, Viskosität, gemessen bei 25 °C, aufweist.

9. Pharmazeutische orale Lösung nach einem der Ansprüche 1 bis 8,
**dadurch gekennzeichnet, dass** die Qualität der Natriumcarboxymethylcellulose so beschaffen ist, dass sie in einer 2 %igen (Gew./Vol.) wässrigen Lösung eine zwischen 25 mPa·s und 2500 mPa·s liegende, vorzugsweise zwischen 400 mPa·s und 800 mPa·s liegende, und insbesondere zwischen 400 mPa·s und 600 mPa·s liegende, Viskosität, gemessen bei 20 °C, aufweist.

10. Pharmazeutische orale Lösung nach einem der Ansprüche 1 bis 9,
**dadurch gekennzeichnet, dass** die Menge an Natriumcarboxymethylcellulose zwischen 2 mg/ml und 25 mg/ml liegt, vorzugsweise zwischen 4 mg/ml und 20 mg/ml liegt, und insbesondere zwischen 6 mg/ml und 14 mg/ml liegt.

11. Pharmazeutische orale Lösung nach einem der Ansprüche 1 bis 10,
**dadurch gekennzeichnet, dass** das Gewichtsverhältnis von niedermolekularem Polyethylenglycol plus Glycerin zu Wasser zwischen 3:1 und 7:1 liegt, vorzugsweise zwischen 4:1 und 6:1 liegt, und insbesondere bei etwa 5:1 liegt.

12. Pharmazeutische orale Lösung nach einem der Ansprüche 1 bis 11,
**dadurch gekennzeichnet, dass** die Lösung ein Süßungsmittel enthält, das vorzugsweise ausgewählt ist aus Aspartam, Kaliumacesulfam, Alitam, Neohesperidin-dihydrochalkon, Neotam, Saccharin, Natriumsaccharin, Sucralose, Thaumatin und Gemischen davon, insbesondere ausgewählt ist aus Saccharin, Natriumsaccharin, Sucralose, Neohesperidin und Gemischen davon, und besonders bevorzugt ein Gemisch aus Sucralose und Neohesperidin ist.

13. Pharmazeutische orale Lösung nach einem der Ansprüche 1 bis 12,
**dadurch gekennzeichnet, dass** sie einen Aromastoff enthält, vorzugsweise in einer zwischen 0,01 mg/ml und 25 mg/ml liegenden Menge.

14. Pharmazeutische orale Lösung nach einem der Ansprüche 1 bis 13 zur therapeutischen Anwendung.

15. Pharmazeutische orale Lösung zur Verwendung nach Anspruch 14,
**dadurch gekennzeichnet, dass** sie zur Behandlung von Fieber und/oder leichten bis mittelschweren Schmerzen, vorzugsweise bei pädiatrischen Patienten, dient.

## Revendications

1. Solution orale pharmaceutique liquide, qui comprend :
a) du paracétamol en une quantité comprise entre 85 mg/ml et 120 mg/ml ;
b) du polyéthylène glycol de faible poids ;
c) de la glycérine ;
d) de la carboxyméthylcellulose sodique ; et
e) de l'eau,
dans lequel la solution orale pharmaceutique présente un rapport pondéral du polyéthylène glycol de faible poids plus la glycérine à l'eau compris entre 2:1 et 8:1, et un rapport pondéral de la glycérine au polyéthylène glycol de faible poids compris entre 1:1 et 2:1.

2. Solution orale pharmaceutique selon la revendication 1, **caractérisée en ce qu'**elle comprend :
a) du paracétamol en une quantité comprise entre 85 mg/ml et 120 mg/ml ;
b) du polyéthylène glycol de faible poids ;
c) de la glycérine ;
d) de la carboxyméthylcellulose sodique ;
e) de l'eau ;
f) un édulcorant ;
g) un agent aromatisant ; et
h) facultativement, un colorant.

3. Solution orale pharmaceutique selon les revendications 1 ou 2, **caractérisée en ce que** la quantité de paracétamol est comprise entre 90 mg/ml et 110 mg/ml, de préférence comprise entre 95 mg/ml et 105 mg/ml, et de manière encore plus préférée est d'environ 100 mg/ml.

4. Solution orale pharmaceutique selon l'une quelconque des revendications 1 à 3, **caractérisée en ce que** le polyéthylène glycol de faible poids présente un poids moléculaire moyen compris entre 400 et 900.

5. Solution orale pharmaceutique selon l'une quelconque des revendications 1 à 4, **caractérisée en ce que** le polyéthylène glycol de faible poids est choisi parmi le PEG 400, le PEG 500, le PEG 600, le PEG, le PEG 700, le PEG 800, le PEG 900 et des mélanges de ceux-ci, et est de préférence le PEG 600.

6. Solution orale pharmaceutique selon l'une quelconque des revendications 1 à 5, **caractérisée en ce que** la teneur totale en polyéthylène glycol de faible poids plus glycérine est de 700 mg/ml à 1100 mg/ml, de préférence de 750 mg/ml à 1000 mg/ml, de manière plus préférée de 800 mg/ml à 950 mg/ml, et de manière encore plus préférée de 850 mg/ml à 900 mg/ml.

7. Solution orale pharmaceutique selon l'une quelconque des revendications 1 à 6, **caractérisée en ce que** le rapport pondéral glycérine:polyéthylène glycol de faible poids est compris entre 1,1:1 et 1,8:1, de préférence compris entre 1,2:1 et 1,6:1, de manière plus préférée compris entre 1,3:1 et 1,5:1, et de manière encore plus préférée est d'environ 1,4:1.

8. Solution orale pharmaceutique selon l'une quelconque des revendications 1 à 7, **caractérisée en ce qu'**elle présente une viscosité comprise entre 3000 mPa.s et 7000 mPa.s, de préférence comprise entre 3500 mPa.s et 6000 mPa.s, mesurée à 25°C.

9. Solution orale pharmaceutique selon l'une quelconque des revendications 1 à 8, **caractérisée en ce que** la qualité de la carboxyméthylcellulose sodique est telle qu'elle procure une viscosité comprise entre 25 mPa.s et 2500 mPa.s, de préférence comprise entre 400 mPa.s et 800 mPa.s, et de manière plus préférée comprise entre 400 mPa.s et 600 mPa.s, en solution aqueuse à 2 % p/v, mesurée à 20°C.

10. Solution orale pharmaceutique selon l'une quelconque des revendications 1 à 9, **caractérisée en ce que** la quantité de carboxyméthylcellulose sodique est comprise entre 2 mg/ml et 25 mg/ml, de préférence comprise entre 4 mg/ml et 20 mg/ml, de manière plus préférée comprise entre 6 mg/ml et 14 mg/ml.

11. Solution orale pharmaceutique selon l'une quelconque des revendications 1 à 10, **caractérisée en ce que** le rapport pondéral du polyéthylène glycol de faible poids plus la glycérine à l'eau est compris entre 3:1 et 7:1, de manière plus préférée compris entre 4:1 et 6:1, et de manière encore plus préférée est d'environ 5:1.

12. Solution orale pharmaceutique selon l'une quelconque des revendications 1 à 11, **caractérisée en ce que** la solution comprend un agent édulcorant, qui est de préférence choisi parmi l'aspartame, l'acésulfame de potassium, l'alitame, la néohespéridine de dihydrochalcone, le néotame, la saccharine, la saccharine de sodium, le sucralose, la thaumatine et des mélanges de ceux-ci, de manière plus préférée est choisi parmi la saccharine, la saccharine de sodium, le sucralose, la néohespéridine et des mélanges de ceux-ci, et de manière plus préférée est un mélange de sucralose et de néohespéridine.

13. Solution orale pharmaceutique selon l'une quelconque des revendications 1 à 12, **caractérisée en ce qu'**elle comprend un agent aromatisant, de préférence en une quantité comprise entre 0,01 mg/ml et 25 mg/ml.

14. Solution orale pharmaceutique liquide selon l'une quelconque des revendications 1 à 13 pour utilisation en thérapie.

15. Solution orale pharmaceutique pour utilisation selon la revendication 14, **caractérisée en ce qu'**elle est destinée au traitement de la fièvre et/ou de la douleur légère à modérée, de préférence chez le patient pédiatrique.
